# EUROPEAN PATENT APPLICATION

(11) **EP 1 510 223 A1**
(43) Date of publication of application: **02.03.2005**
(21) Application number: 03733078.4
(22) Date of filing: 27.05.2003
(51) Int. Cl.: A61K 47/34, A61K 9/14, A61K 31/5575, A61K 31/573, A61K 47/24, A61K 47/26, A61P 9/14, A61P 43/00

(54) **INTRAVENOUS COMPOSITION, PROCESS FOR PRODUCING THE SAME AND PREPARATION THEREOF**

(30) Priority: 31.05.2002 JP 2002159190
(71) Applicant: LTT Bio-Pharma Co., Ltd., Tokyo 105-6201 (JP)
(72) Inventor: MIZUSHIMA, Yutaka, Tokyo 106-0032 (JP); ISHIHARA, Tsutomu, Ota-ku, Tokyo 143-0024 (JP)
(74) Representative: Rupp, Christian, Dipl.Phys.
(86) International application number: PCT/JP2003/006571
(87) International publication number: WO 2003/101493

(57) **Abstract**

A composition for intravenous injection, which is gradually decomposed instead of fat particles, has sufficient sustained release effects, has an excellent encapsulation ratio of lipid-soluble agents, and has such sustained release effects at lesion sites; a production method thereof; and a preparation containing the composition.

The composition for intravenous injection is produced by encapsulating a prostanoid or steroid in a poly(lactic-co-glycolic acid) or poly(lactic acid) microparticle, and allowing lecithin or similar surfactant to be adsorbed on the surface of the above poly(lactic-co-glycolic acid) or poly(lactic acid) microparticle. A method for producing the composition comprises: dissolving esterified prostanoic acid or esterified steroid and a poly(lactic-co-glycolic acid) or poly(lactic acid) in an organic solvent such as dichloromethane or dimethylsulfoxide; and emulsifying the obtained mixture in water, using the lecithin or similar surfactant capable of adjusting the diameter of the copolymer or particle to between 50 and 500 nm, employing an ultrasonic generator, a Polytron Homogenizer, or the like.

## Description

### BACKGROUND OF THE INVENTION

### Field of the Invention

The present invention relates to a composition for intravenous injection, a production method thereof, and a preparation containing the composition. More specifically, the present invention relates to a composition for intravenous injection that is used for the purpose of targeting an agent to a lesion site and the sustained release of the agent, a production method thereof, and a preparation containing the composition.

### Description of the Related Art

One of the present inventors has previously developed vascular wall targeting agents (lipo PGE1) comprising prostaglandin E1 (PGE1) or prostanoids similar thereto. These agents have been produced by encapsulating PGE1 or an ester thereof in a fatty particle with a diameter of 200 nm. Some of these agents have been commercially available and have been widely used in several countries.

The second generation lipo PGE1 in which PGE1 ester is encapsulated is excellent instability and encapsulation ratio, and the effects of the second generation agent are considerably higher than those of the first generation agent.

Among patents submitted by other inventors, there are some patents in which PGE1 or PGI2 selected from among a large number of agents is encapsulated in a poly (lactic-co-glycolic acid)(PLGA) or poly(lactic acid)(PLA) microparticle and it is then applied to a drug delivery system (DDS).

However, since the carrier for the aforementioned lipo PGE1 is easily decomposable, there has been a problem in that the lipo PGE1 is decomposed immediately after it is targeted to the vascular wall, and that the principal agent is released, meaning that sufficient sustained release effects cannot be obtained.

In addition, the technique of encapsulating the aforementioned PGE1 or PGI2 in PLGA and applying the obtained product to DDS has also been problematic in that PGE1 or PGI2 per se is not encapsulated in the PLGA particle.

The present inventors have considered even a particle's encapsulation ratio or drug kinetics *in vivo,* and have studied the most suitable ester form of prostanoids such as PGE1. In our previous invention, the surface of a particle with a diameter of 200 nm was covered with lecithin. However, the present inventors have reviewed the particle size or surfactants, and have studied to establish a production method. At the same time, they have studied the most suitable ester form of steroids. From these studies, the present inventors have provided the most excellent sustained-release targeting product, that is, a prostanoid PLGA/PLA product or steroid PLGA/PLA product.

Thus, it is an object of the present invention to provide: a composition for intravenous injection, which can be gradually decomposed instead of lipo particles, has sufficient sustained release effects, has the same excellent encapsulation ratio as that of the second generation lipo PGE1, and has sustained release effects at lesion sites; a production method thereof; and a preparation containing the composition.

### SUMMARY OF THE INVENTION

In order to achieve the aforementioned object, the composition for intravenousinjection ofthe present invention comprises, PLGA or PLA microparticle in which the prostanoid or steroid is encapsulated, and which is adsorbed by a lecithin or similar surfactant on the surface of the PLGA or PLA microparticle.

In order that PLGA or PLA easily accumulate a lesion site, the diameter of the PLGA or PLA microparticle is preferably between 50 and 500 nm. When the diameter of the particle is less than 50 nm, it is so small that it enters sites other than the lesion site. In contrast, when the diameter of the particle is from 500 nm to 1,000 nm, or greater, it is so large that it cannot accumulate the lesion site.

The above prostanoid is preferably a prostanoic acid ester-type prodrug, which is easily encapsulated in a PLGA or PLA microparticle, is chemically stable, and is easily converted into prostanoic acid *in vivo.*

The above prostanoic acid ester-type prodrug is preferably formed by introducing alkyl ester (C1-C10) into position 1 of PGE1 and introducing an acyl group (C2-C5) into position 9 thereof.

The above steroid is preferably a steroid ester.

The above steroid ester is preferably formed by introducing acyl ester (C2-C10) into positions 17 and 21 of betamethazone or a steroid similar thereto.

The method of the present invention for producing a composition for intravenous injection comprises: dissolving an esterified prostanoic acid or esterified steroid and a PLGA or PLA microparticle in an organic solvent including, as a typical example, dichloromethane or dimethylsulfoxide (DMSO); and emulsifying the obtained mixture in water, using a lecithin or similar surfactant (Pluronic, etc.) capable of adjusting the diameter of the above particle to between 50 and 500 nm, employing an ultrasonic generator, a Polytron Homogenizer, or the like.

Moreover, the method of the present invention for producing a composition for intravenous injection further comprises: removing lecithin or similar surfactant (in which a large amount of principal agent may be contained) from a composition produced by the above-described production method; adding a lecithin or a micelle of similar surfactant to a water suspension containing the composition; and allowing the lecithin or similar surfactant to be adsorbed again on the surface of the PLGA or PLA particle.

Furthermore, the preparation for intravenous injection of the present invention comprises a stabilizer and an isotonizing agent, which are used to resuspend the freeze-dried composition for intravenous injection before administration.

Further, the composition for intravenous injection of the present invention is produced by encapsulating, in a PLGA or PLA microparticle, an agent capable of sustained release in a cell such as macrophage with phagocytotic action, while maintaining biological activity.

As stated above, the composition for intravenous injection of the present invention, that is, an agent-encapsulated preparation, consists of a PLGA/PLA particle covered with a surfactant such as lecithin and prostanoic acid ester or steroid ester. In order to utilize this microparticle as a preparation, it is important to control the surface properties, particle size, agent encapsulation ratio, and releasing behavior of the particle.

Surface properties can be controlled using lecithin or surfactants similar thereto. In addition, since a surfactant affects an emulsification state, the weight ratio between lecithin and a PLGA/PLA microparticle is varied when the composition is prepared, or the strength of an emulsifier such as an ultrasonic generator is changed, so as to control the particle size. Particles accumulate different types of lesion sites (pathologic vascular wall, inflammatory sites, carcinoma tissues, and the reticuloendothelial system involved in formation of a pathologic state), depending on particle size.

In order to increase the encapsulation ratio of an agent in a preparation, the agent is esterified to increase its fat solubility. In addition, it is necessary to optimize the length of an alkyl or acyl chain to be introduced during the esterification. Further, the use of PLGA or PLA microparticles having different molecular weights enables the control of the sustained-release rate of the agent. In order to evaluate the developed preparation, it is necessary to construct an *in vitro* or animal (*in vivo*) model, which is suitable for the analysis of PK/PD (drug kinetics or pharmacological action).

As stated above, in the present invention, gradually decomposed PLGA/PLA microparticles are used instead of lipo particles, and the microparticles have sufficient sustained release effects. Moreover, the use of an esterified steroid enables the production of a preparation, which has the same excellent encapsulation ratio as that of the second generation lipo PGE1 and has sustained release effects at a lesion site; that is, a preparation having both excellent targeting effects and sustained release effects.

### BRIEF DESCRIPTION OF THE DRAWINGS

FIG. 1 is a view showing the encapsulation ratio of various agents in the PLGA microparticle produced in Example 1;
FIG. 2 is a view showing the releasing behavior of AS006 from the AS006-encapsulated PLGA micromicroparticle produced in Example 2;
FIG. 3 is a view showing the releasing behavior of BDP from the BDP-encapsulated PLGA microparticle produced in Example 2;
FIG. 4 is a view showing the remaining ratios of various agents in various-agent-encapsulated PLGA microparticles, which were obtained after the various-agent-encapsulated PLGA microparticle produced in Example 1 was suspended and left in a 1% SDS aqueous solution or 80% FBS at 37°C for 5 minutes;
FIG. 5 is a view showing the turbidity of a PLGA microparticle obtained after the PLGA microparticle produced in Example 2 was freeze-dried in the presence of various stabilizers and was then resuspended in water;
FIG. 6 is a view showing the releasing behavior of AS006, which was observed after the PLGA particle produced in Example 4, from which AS006 on the surface layer was removed, was freeze-dried in 10% sucrose and was then resuspended in 3% BSA-containing PBS;
FIG. 7 is a view showing the releasing behavior of BDP, which was observed after the BDP-encapsulated PLGA microparticle produced in Example 2 was freeze-dried in 10% sucrose and was then resuspended in 3% BSA-containing PBS;
FIG. 8 is a view showing the uptake of the rhodamine-encapsulated PLGA microparticle produced in Example 1 in a macrophage cell and the residual tendency of the rhodamine uptaken by the cell that was observed over time with a fluorescent microscope; and
FIG. 9 is a view showing the inflammation inhibitory effects of the BDP-containing PLGA microparticle produced in Example 2 on an arthritis model rat.

### DETAILED DESCRIPTION OF THE PREFERRED EMBODIMENTS

The examples of the present invention will be described below.

### (Example 1) Production method of PLGA/PLA preparation

100 mg of PLGA (Wako) or PLA (Wako), 10 mg of egg yolk lecithin, and 0.5 mg of an agent were dissolved in 1 ml of dichloromethane. While cooling in an ice bath, the obtained solution was slowly added dropwise, through a 27G needle, to 25 ml of distilled water, which was being stirred with Polytron PT-2100 (Kinematica) or an ultrasonic generator (TOMY). After the stirring was continued for 10 minutes, the mixture was further stirred with a stirrer at room temperature for 2 hours. Thereafter, dichloromethane was removed. The obtained microparticles were concentrated by ultrafiltration (Amicon, Centriprep YM-10) and were then purified by gel filtration (Pharmacia, PD-10). The thus obtained particles were centrifuged at 13,000 g for 10 minutes, and the agent contained in the supernatant and deposit was assayed by HPLC. HPLC analysis was carried out in a water/acetonitrile system for measuring the absorption at 210 nm or 240 nm using a C4 reverse phase column (Waters, Symmetry 300). With regard to agents, PGE1, and AS006 produced by esterifying carboxylic acid and a hydroxyl group thereof, were used as prostanoids. Hydrocortisone, hydrocortisone acetate, hydrocortisone butyrate propionate (HBP), betamethazone, and betamethazone dipropionate (BDP) were used as steroids.

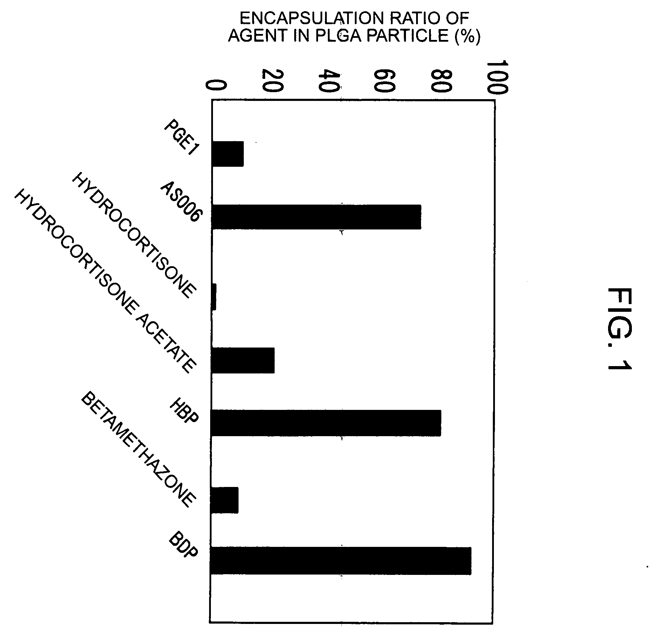

As shown in FIG. 1, as a result, it was found that almost no PGE1 could be encapsulated, but that the encapsulation ratio of AS006 as an ester form thereof was significantly increased. It was also found that almost neither hydrocortisone nor betamethazone could be encapsulated, but that the encapsulation ratio of HBP and BDP as ester forms thereof was significantly increased.

### (Example 2)

30 mg of PLGA (Wako) or PLA (Wako), 3 mg of egg yolk lecithin (Wako), and 1 mg of AS006 or BDP were dissolved in 1 ml of dichloromethane, and microparticles were then produced by the same method as described in Example 1. The obtained microparticles were suspended in distilled water, PBS, or 3% BSA-containing PBS. The suspension was allowed for a certain period of time, and was then centrifuged at 13,000 g for 10 minutes. Thereafter, AS006 or BDP contained in the deposit was assayed by HPLC.

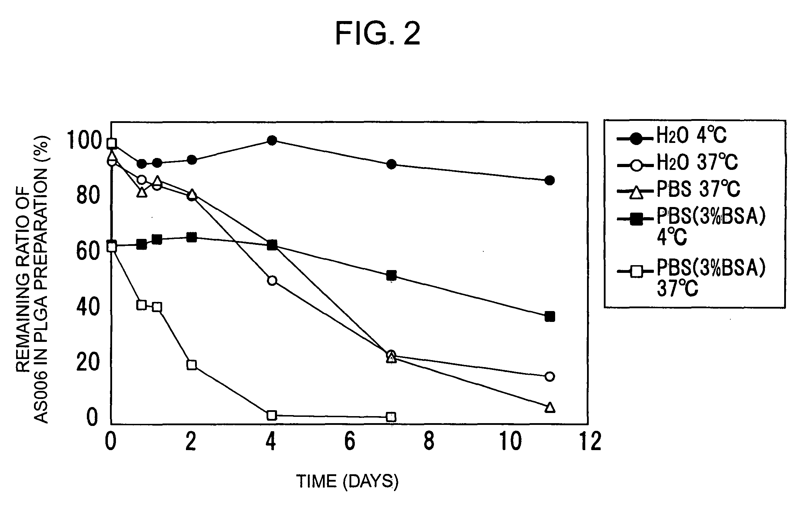

As shown in FIG. 2, as a result, it was found that AS006 was gradually released over 11 days in the water or PBS at 37°C, and that AS006 was released over 4 days in the 3% BSA-containing PBS at 37°C. Thus, it became clear that prostanoid was released from the microparticles.

In a case where the microparticles were incubated in water at 4°C, even after 10 days, almost no release of AS006 was observed. Further, in 3% BSA-containing PBS at 4°C, approximately 30% of the agent was once released at the initial stage, but thereafter, extremely slow releasing behavior was observed. Thus, it became clear that these microparticles are stable even in water suspension at 4°C.

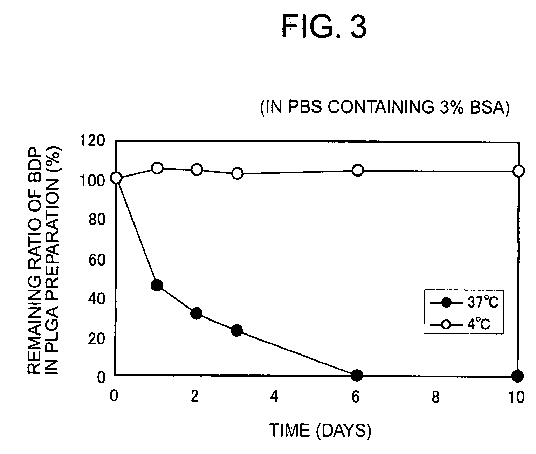

Furthermore, as shown in FIG. 3, it was found that BDP exhibits the same sustained-release behavior as that of AS006 in 3% BSA-containing PBS at 37°C.

### (Example 3)

Each of the agents produced in Example 1 was suspended in 80% FBS (fetal bovine serum) or in a 1% SDS aqueous solution, and the suspension was incubated at 37°C for 5 minutes. Thereafter, the resultant product was centrifuged at 13,000 g for 10 minutes, and the amount of the agent contained in the deposit was assayed by HPLC.

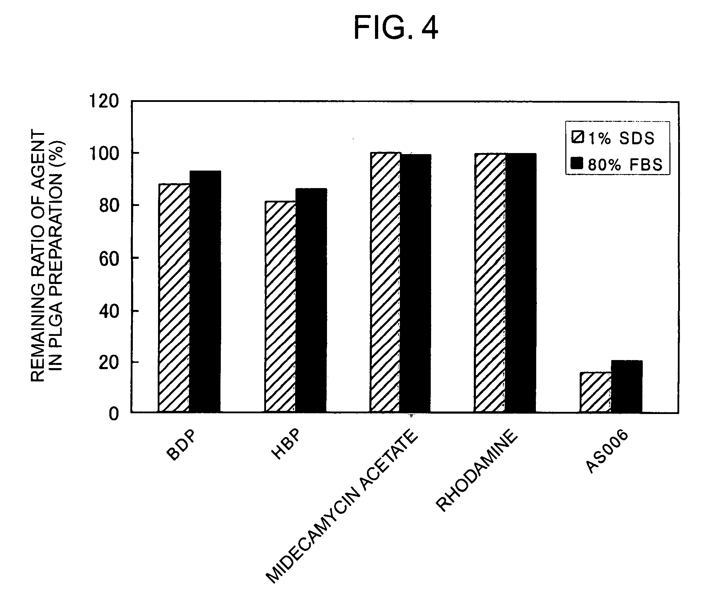

As shown in FIG. 4, as a result, it was found that only 10% to 20% AS006 remained in the presence of 1% SDS or in 80% FBS. Although the production conditions such as the weight ratio of PLGA to AS006 were increased, the ratio of the distributionwasnotchanged. Thus, itbecameclearthatAS006 has a higher affinity to the lecithin layer than to the PLGA layer. Moreover, it was also found that the remaining ratio of AS006 is reduced in a BSA-concentration-dependent manner. On the other hand, in the case of BDP as a steroid derivative or other agents, 80% or more agents remained in PLGA even with the addition of SDS, and thus, it became clear that the agent was distributed in PLGA.

### (Example 4)

The AS006-encapsulated PLGA microparticles produced in Example 2 were suspended in 1% SDS, and the suspension was incubated at 37°C for 5 minutes. Thereafter, the resultant product was washed by centrifugation at 5,000 g for 10 minutes. While applying ultrasonic waves, the obtained microparticles were dispersed again in a lecithin suspension. Thereafter, the surface potential (zeta potential) value of the microparticles was measured.

The surface potential value of the preparation produced in Example 2 was -6.6 mV. After addition of SDS, however, the value became -57.2 mV. Thus, the value experienced significant negative change. As a result, it became clear thatlecithinhadbeendesorbedfromthesurface. Inaddition, as described in Example 3, AS006 existing in the surface layer was also released from the microparticles at the same time. Moreover, when the microparticles were treated with ultrasonic waves in a lecithin suspension after washing them by centrifugation, the surface potential value became -6.3 mV, which was almost the same value as that before addition of SDS. Accordingly, it was clarified that PLGAmicroparticles, the surfaces of which were covered with lecithin and in which AS006 was encapsulated, could be produced.

### (Example 5)

A 10% stabilizer was added to the PLGA microparticle suspension produced in Example 2, and the suspension was then frozen with acetone/dry ice, followed by performing a freeze-drying treatment. The dried PLGA microparticles were dispersed in water again. Thereafter, the turbidity of the suspension was measured with a spectrophotometer, and the microparticle size thereof was measured with a dynamic light scattering photometer. In addition, the AS006-containing PLGA microparticles produced in Example 4 and the BDP-containing PLGAmicroparticles produced in Example 2 were freeze-dried in 10% sucrose, and the remaining amount of the agent and the releasing behavior were measured by the method described in Example 2.

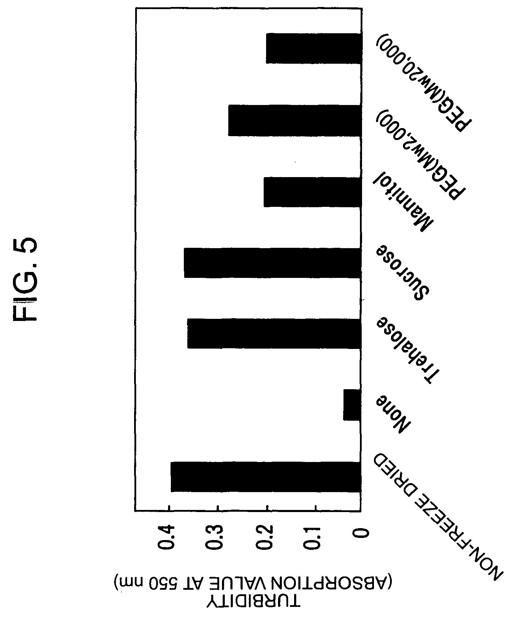

As shown in FIG. 5, as a result, in the PLGAmicroparticles that were freeze-dried only with water (no additives), a decrease in the turbidity due to the formation of aggregates was observed, and thus, redispersibility was significantly low. Even in a case where mannitol or PEG was added as a stabilizer, aggregationwas obs erved to a considerable extent. However, when trehalose or sucrose was added, almost the same level of turbidity as that before the freeze-drying treatment was maintained. The particle size of these particles was measured by the light-scattering method. As a result, the mean microparticle size (mean weight value) of particles that had not been subjected to a freeze-drying treatment, was found to be 319 nm, whereas those of microparticles, to which trehalose and sucrose had been added were found to be 381 nm and 398 nm, respectively. Thus, it was found that microparticles with high redispersibility were selected although a small degree of aggregation was still observed.

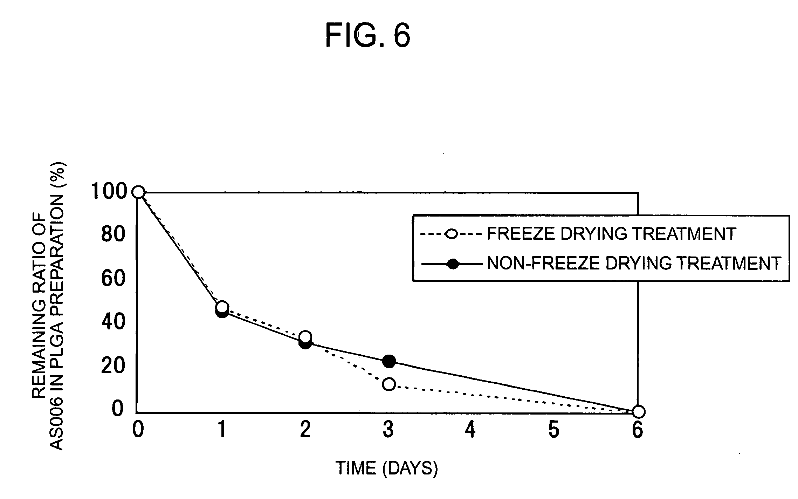

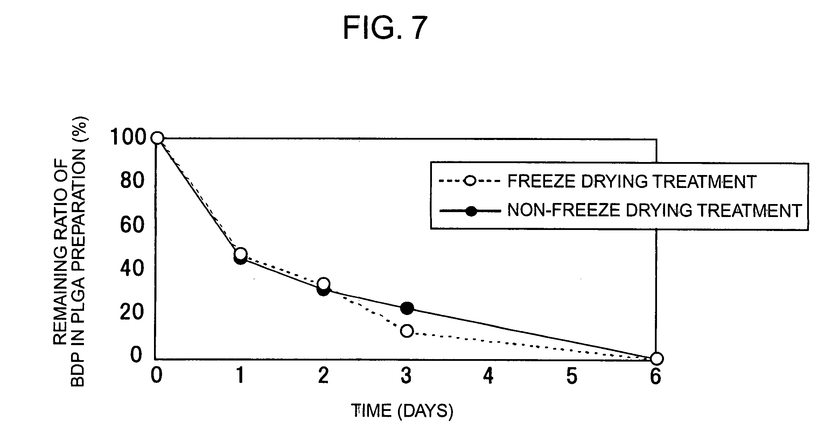

Moreover, the releasing behavior of AS006 and BDP in 3% BSA-containing PBS was examined. As a result, as shown in FIGS. 6 and 7, both agents exhibited the same releasing behavior regardless whether or not a freeze-drying treatment had been carried out, and thus, it was clarified that such a freeze-drying treatment does not affect the sustained release of the agent.

### (Example 6)

Macrophages were collected from the abdominal cavity of a mouse, which had been stimulated by administration of 1.5 ml of 10% proteose peptone. The collected macrophages were inoculated with a concentration of 100, 000 cells/48 wells, and they were then cultured in an RPMI1640 medium (containing 10% FBS) for several days. After exchanging the medium with a fresh medium, PLGA microparticles, in which rhodamine, a fluorescent dye produced by the method described in Example 1, had been encapsulated as an agent model, were added thereto, followed by incubation at 37°C for 1.5 hours. The resultant product was washed with PBS 3 times and was then left for a certain period of time. Thereafter, the cells were immobilized with a 4% neutral formalin solution, and were then observed with a fluorescent microscope (IX-70, Olympus).

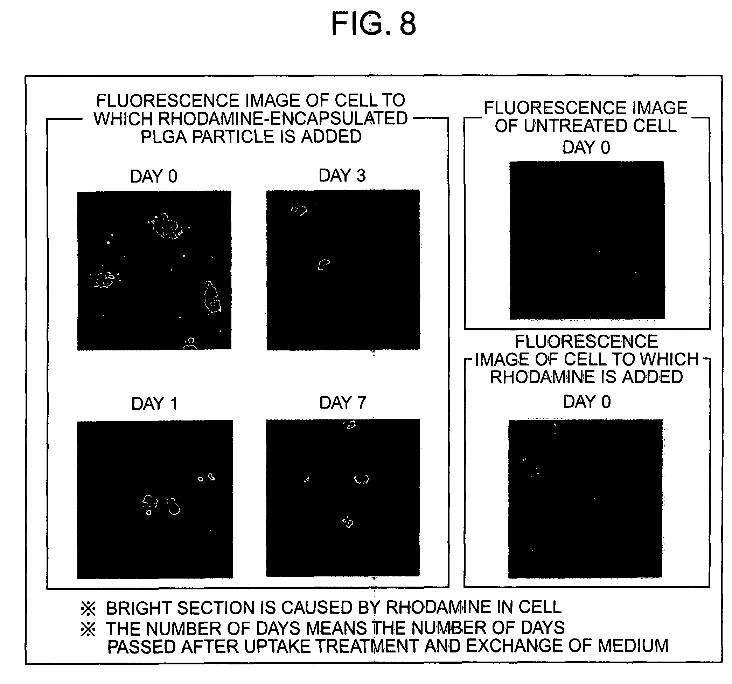

As shown in FIG. 8, as a result, it was found that rhodamine encapsulated in microparticles was internalized macrophages at a higher level than a case where rhodamine alone was added. After the microparticles was internalized the macrophages, the medium was exchanged with a fresh medium, followed by incubation at 37°C. As a result, it was found that a considerable amount of rhodamine remained in the cells even after 1 week.

Moreover, the same studies were carried out, with the difference that PLA microparticles were used instead of PLGA microparticles, and the same results were obtained.

### (Example 7)

100 µl of an adjuvant suspension containing 6 mg/ml dry M. Butyricum was intravenously injected through the tail head portion of a rat. 6 days later, 100 µl of a physiological salt solution containing 1% carrageenin was injected into the sole of the left foot thereof, so as to produce a continued arthritis model rat (Y. Mizushima et al., J. Pharm. Pharmac., 1972, 24, 781-785). At 24 hours after administration of carrageenin, the BDP-containing PLGA preparation produced in Example 2 (50 µg/rat betamethazone) was injected into the caudal vein thereof, and the swelling of the foot of the rat was measured with a volume meter over time. As a control, there was used a rat into which betamethazone phosphate was subcutaneously injected, having the same titer as that of a rat into the caudal vein of which PBS alone was injected.

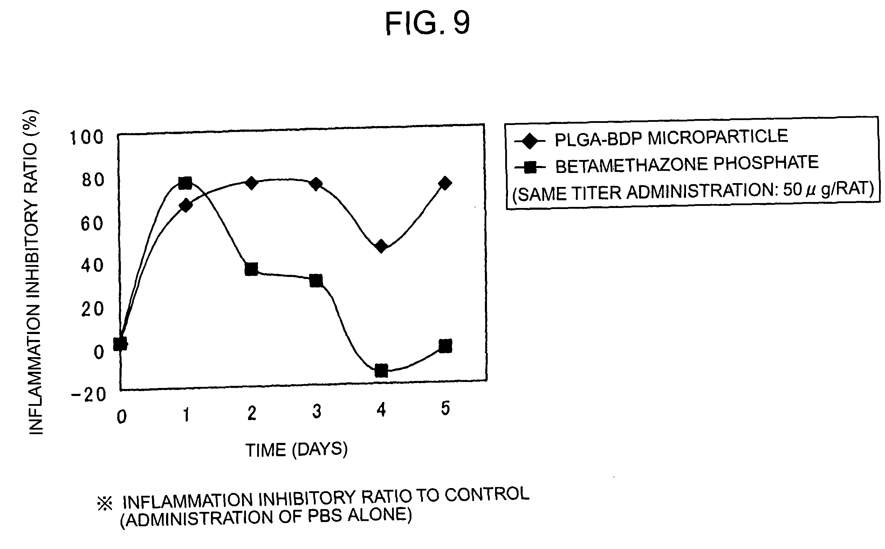

As shown in FIG. 9, as a result, it was found that when betamethazone phosphate was administered, swelling was strongly inhibited on the 1^{st} day, but thereafter, such inhibitory effects rapidly decreased. In contrast, when the BDP-containing PLGA preparation was administered, the same level of inhibitory effects as those of betamethazone phosphate were shown on the 1^{st} day, and thereafter, swelling was strongly inhibited over several days.

## Claims

1. A composition for intravenous injection, comprising a poly(lactic-co-glycolic acid) or poly(lactic acid) microparticle in which the prostanoid or steroid is encapsulated, and which is adsorbed by a lecithin or similar surfactant on the surface thereof.

2. The composition for intravenous injection according to claim 1, wherein the poly(lactic-co-glycolic acid) or poly(lactic acid) microparticle has a diameter between 50 and 500 nm.

3. The composition for intravenous injection according to claim 1, wherein the prostanoid is a prostanoic acid ester-type prodrug, which is easily encapsulated in a poly(lactic -co-glycolic acid) or poly(lactic acid) microparticle, is chemically stable, and is easily converted into prostanoic acid *in vivo.*

4. The composition for intravenous injection according to claim 1 or 3, wherein the prostanoic acid ester-type prodrug is formed by introducing an alkyl ester into position 1 of prostaglandin E1 and introducing an acyl group into position 9 thereof.

5. The composition for intravenous injection according to claim 1, wherein the steroid is a steroid ester.

6. The composition for intravenous injection according to claim 1 or 5, wherein the steroid ester is formed by introducing an acyl ester into positions 17 and 21 of betamethazone or a similar steroid.

7. A method for producing a composition for intravenous injection, comprising: dissolving an esterified prostanoic acidor esterified steroid and a poly(lactic-co-glycolic acid) or poly(lactic acid) in an organic solvent including, as a typical example, dichloromethane or dimethylsulfoxide; and emulsifying the obtained mixture in water, using a lecithin or similar surfactant capable of adjusting the diameter of the copolymer or particle to between 50 and 500 nm.

8. A method for producing a composition for intravenous injection, comprising: removing a lecithin or similar surfactant from a composition produced by the production method according to claim 7; adding a lecithin or a micelle of the similar surfactant to a water suspension containing the composition; and allowing the lecithin or similar surfactant to be adsorbed again on the surface of the poly(lactic-co-glycolic acid) or poly(lactic acid) microparticle.

9. A preparation for intravenous injection, comprising the composition for intravenous injection according to claim 1 and a stabilizer and an isotonizing agent both which are added to resuspend the freeze-dried composition for intravenous injection before administration.

10. The preparation for intravenous injection according to claim 9, wherein the stabilizer is trehalose or sucrose.

11. The composition for intravenous injection according to claim 1, which is produced by encapsulating, in a poly(lactic-co-glycolic acid) or poly(lactic acid) microparticle, an agent capable of sustained release in a cell such as macrophage with phagocytotic action, while maintaining biological activity.
